# EUROPÄISCHE PATENTANMELDUNG

(11) **EP 2 399 639 A1**
(43) Veröffentlichungstag der Anmeldung: **28.12.2011**
(21) Anmeldenummer: 10075277.3
(22) Anmeldetag: 25.06.2010
(51) Int. Cl.: A61M 25/01, A61M 25/06, A61M 1/10

(54) **System zum einführen einer pumpe**

(71) Anmelder: ECP Entwicklungsgesellschaft mbH, 12247 Berlin (DE)
(72) Erfinder: Die Erfindernennung liegt noch nicht vor
(74) Vertreter: Pfenning, Meinig & Partner GbR

(57) **Zusammenfassung**

Die Erfindung liegt auf dem Gebiet des Einführens von Fluidpumpen in ein Lumen und betrifft ein System zum Einführen einer Pumpe in ein Lumen, welches eine erste Schleuse und eine in die erste Schleuse einzuführende Pumpe umfasst, bzw. ein System, welches eine Pumpe mit einer distalen Pumpeneinheit und einem proximal der Pumpeneinheit austretenden Wellenkatheter aufweist.

Erfindungsgemäß werden ein oder zwei Schleusen verwendet, wobei die distale Pumpeneinheit zunächst in das distale Ende einer Schleuse eingezogen wird, um Beschädigungen eines Wellenkatheters zu vermeiden. Anschließend wird die die Pumpeneinheit aufnehmende Schleuse in eine weitere Schleuse oder ein aufnehmendes Lumen überführt.

## Beschreibung

Die Erfindung liegt auf dem Gebiet des Einführens von Fluidpumpen in ein Lumen und betrifft ein System zum Einführen einer Pumpe in ein Lumen, welches eine erste Schleuse und eine in die erste Schleuse einzuführende Pumpe umfasst, bzw. ein System, welches eine Pumpe mit einer distalen Pumpeneinheit und einem proximal der Pumpeneinheit austretenden Wellenkatheter aufweist.

Die Anwendung kann somit einerseits im minimalinvasiven medizinischen Bereich, beispielsweise als Blutpumpe zur Herzunterstützung, vorgesehen sein, andererseits ist auch ein Einsatz in Rührwerken oder als Vortriebselement denkbar.

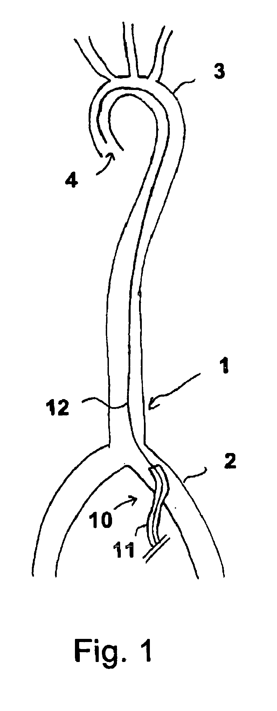

Besondere Vorteile kann die Erfindung durch die mögliche Miniaturisierung im medizinischen Bereich entfalten. Techniken zum Einführen von Fluidpumpen, insbesondere in körpereigene Lumina, sind im Stand der Technik ausführlich bekannt. So wird unter anderem auf die Seldinger-Technik zum Einführen einer Einführschleuse in ein Gefäßsystem verwiesen. Die Technik und etwaige Varianten sollen anhand der Fig. 1 genauer erläutert werden.

In der Fig. 1 ist ein schematisches menschliches Gefäßsystem 1 gezeigt. Im Bereich der Leiste liegt eine der femoralen Arterien 2, die über eine Hauptschlagader mit dem Aortenbogen 3 verbunden ist und anschließend in die Herzkammer 4 mündet. Mithilfe beispielsweise der Seldinger-Technik wird zunächst eine Einführschleuse 10 in die femorale Arterie 2 eingeführt. Hierbei wird zunächst die femorale Arterie bzw. ein beliebiges Blutgefäß beispielsweise mit einer Stahlkanüle mit schneidender Spitze punktiert. Durch die in die Punktur eingeführte Stahlkanüle hindurch wird ein Führungsdraht 12 geschoben und retrograd über den Aortenbogen 3 in den linken Herzventrikel 4 eingeführt. Nach Entfernung der Punktionskanüle wird die als Einführschleuse ausgebildete erste Schleuse 10, welche einen schlauchförmigen Abschnitt 11 umfasst, auf den Führungsdraht eingefädelt und durch die punktierte Stelle in das Gefäßsystem eingeführt, wobei die Schleuse nur um eine geringe Distanz in das Lumen des Gefäßsystems eingeführt wird. Anschließend wird eine Fluidpumpe durch die Einführschleuse 10 in das Gefäßsystem eingeführt.

Ein derartiges Verfahren ist beispielsweise aus der WO 02/43791 A1 bekannt. Nachdem die Einführschleuse, wie in der Fig. 1 darqestellt, in das Gefäßsystem eingeführt wurde, wird eine Herzpumpe entlang des Führungsdrahtes 12 bis in den linken Herzventrikel 4 vorgeschoben. Die Pumpe, insbesondere eine distale Pumpeneinheit, wird dabei außerhalb der Schleuse durch das Gefäßsystem zum Ventrikel geschoben.

Moderne Fluidpumpen drehen oftmals mit hohen Drehzahlen, um eine entsprechende Menge an Fluid pro Minute fördern zu können. Eine derartige Blutpumpe ist beispielsweise der vorgenannten WO 02/43791 A1 oder der EP 2 047 872 A1 entnehmbar. Letztere zeigt eine Pumpe, welche über eine distale Pumpeneinheit verfügt, wobei sich an das proximale Ende der distalen Pumpeneinheit ein Wellenkatheter anschließt, wobei der Wellenkatheter wiederum mit einer Antriebseinheit zum Antrieb der Fluidpumpe verbunden werden kann.

Ein Nachteil an den bislang bekannten Systemen zum Einführen einer Pumpe in ein Lumen ist die große Gefahr, die in dem Wellenkatheter verlaufende Welle zu beschädigen, was sich negativ auf die Lebensdauer bzw. Einsatzdauer der Pumpeinheit auswirkt.

Ausgehend vom bekannten,Stand der Technik ist es die Aufgabe der vorliegenden Erfindung, die Gefahr einer Beschädigung des Wellenkatheters einer Pumpe zu verringern.

Die Aufgabe wird durch ein System nach den Merkmalen des Anspruchs 1 bzw. mithilfe eines Systems nach den Merkmalen des Anspruchs 12 gelöst. Weiterhin werden durch die Erfindung Verfahren zum Einführen und Ausführen von Pumpen umfasst.

Weitere Ausführungsformen sind in den unter- und nebengeordneten Ansprüchen aufgeführt.

Ein erster Aspekt der Erfindung wird durch ein System zum Einführen einer (vorzugsweise expandierbaren) Pumpe (besonders vorzugsweise selbstexpandierbaren Rotationspumpe) in ein Lumen gegeben, wobei das System eine erste Schleuse und eine in die erste Schleuse einzuführende Pumpe umfasst und die erste Schleuse ein erstes, sich von einem distalen Ende zu einem proximalen Ende hin erstreckendes Schleusenlumen zur Führung der Pumpe aufweist.

Erfindungsgemäß umfasst das System ferner eine zweite Schleuse mit einem zweiten, sich von einem distalen Ende zu einem proximalen Ende hin erstreckenden Schleusenlumen, in welchem die Pumpe führbar gehalten ist. Die zweite Schleuse ist dabei derart ausgelegt, dass sie zur Überführung der Pumpe von der zweiten in die erste Schleuse mit der ersten Schleuse koppelbar ist.

Die zweite Schleuse bietet zusätzlichen Schutz für den Wellenkatheter beim Einführen der Pumpe. Auf diese Weise wird ein verbessertes Einführen der Pumpe in die erste Schleuse bzw. in das erste Schleusenlumen ermöglicht.

Im Sinne der vorliegenden Erfindung bedeutet "Schleuse" einen Gegenstand, durch den etwas anderes geführt werden kann. Eine solche Schleuse kann, aber muss nicht zwingenderweise, ein hämostatisches Ventil aufweisen.

Da das Einfädeln der Pumpe in eine Schleuse zumeist mit großer Sorgfalt und damit verbunden einem beträchtlichen Zeitaufwand verbunden ist, ermöglicht die zweite Schleuse ein Einfädeln der Pumpeneinheit in dieselbige vor dem eigentlichen Überführen der Pumpe in das die Pumpe aufnehmende Lumen. Mit anderen Worten kann die Pumpe weit vor einer Operation zunächst inspiziert und anschließend in die zweite Schleuse eingefädelt, vorzugsweise eingezogen werden, wobei das Einfädeln ohne Zeitdruck vorgenommen werden kann.

Zum Zeitpunkt der Operation muss nunmehr die zweite Schleuse, in welcher die Pumpe zunächst gehalten ist, nur an die erste Schleuse angekoppelt werden, und die Pumpe kann direkt von der zweiten in die erste Schleuse überführt werden. Dies verringert die Wahrscheinlichkeit einer Beschädigung der Pumpe bzw. der Welle und ermöglicht so eine verbesserte Laufleistung bzw. Laufzeit der Pumpe selbst. Auch wird der während beispielsweise einer Operation benötigte Zeitaufwand zum Einführen der Pumpe reduziert.

Ein zweiter Aspekt der Erfindung betrifft ein in dem vorgenannten System einsetzbares System mit einer Pumpe, welche eine distale Pumpeneinheit und einen proximal der Pumpeneinheit austretenden Wellenkatheter umfasst. Nebst der Pumpe weist das System ferner eine proximal der distalen Pumpeneinheit angeordnete Schleuse mit einem Schleusenlumen auf, wobei das Schleusenlumen einen Abschnitt des Wellenkatheters umschließt. Das hier lediglich als Schleuse titulierte Merkmal wird im System des ersten Aspekts der Erfindung durchgängig als zweite Schleuse bezeichnet. Derartige Merkmale beziehen sich auch auf das System gemäß dem zweiten Aspekt.

Die Schleuse ist derart ausgebildet, dass die Schleuse entlang des Wellenkatheters in Richtung der distalen Pumpeneinheit verschiebbar ist und die distale Pumpeneinheit in das Schleusenlumen einführbar ist. Dies setzt voraus, dass der Innendurchmesser des Schleusenlumens größer ist als der Außendurchmesser des Wellenkatheters.

An dem aus der Pumpe und der Schleuse bestehenden System ist es insbesondere vorteilhaft, dass zum Einziehen der distalen Pumpeneinheit in das distale Ende der Schleuse am Wellenkatheter gezogen werden kann. Da beim Ziehen der Welle die Gefahr von Verbiegungen oder Beschädigungen oder Abknicken der Welle gegenüber einem Eindrücken bzw. Einschieben der Vorrichtung in eine Schleuse deutlich verringert ist, wird die Laufzeit bzw. Lebensdauer der Pumpeneinheit gegenüber den herkömmlichen Verfahren zum Einführen der Pumpe und den dort eingesetzten Systemen verbessert.

Nachdem die Pumpe in die Schleuse eingezogen wurde und das distale Ende der distalen Pumpeneinheit teilweise oder komplett proximal des distalen Endes der Schleuse liegt, kann das System des zweiten Aspekts gemäß dem ersten Aspekt der Erfindung mit einer ersten Schleuse gekoppelt werden, so dass ein einfacheres Einführen der Pumpeneinheit in das die Pumpe letztendlich aufnehmende Lumen, wie z. B. ein Blutgefäß, erreicht wird.

Gemäß einem dritten Aspekt der Erfindung können die Systeme des ersten oder zweiten Aspekts der Erfindung weiter verbessert werden. Der dritte Aspekt umfasst eine Vorrichtung zum Halten eines Abschnitts der Schleuse des zweiten Aspekts bzw. der zweiten Schleuse des ersten Aspekts. Die Vorrichtung zum Halten des Abschnitts der Schleuse ist dabei derart ausgebildet, dass sie die Schleuse zum einen kraftschlüssig halten kann und zum anderen eine Verformung, insbesondere ein Abknicken, der Schleuse im gehaltenen Bereich verhindert. Durch die Vorrichtung zum Halten eines Abschnitts der Schleuse ist eine gegebenenfalls vorhandene intrinsische Versteifung der Schleuse, beispielsweise in Form eines Drahtgeflechts, nicht nötig, da die Vorrichtung zum Halten eine Versteifung eines Abschnitts der Schleuse bewirkt. Dies führt dazu, dass ein Abknicken der Welle beim Einziehen der Pumpe in das Lumen der Schleuse nicht möglich und beim Überführen der Pumpe von der mittels der Vorrichtung gehaltenen Schleuse in die erste Schleuse erschwert möglich ist.

Die in den vorhergehenden Abschnitten beschriebenen drei Aspekte bilden jeder für sich genommen einen eigenständig patentierbaren Teil der Erfindung. Obgleich der dritte Aspekt, d. h. die Vorrichtung zum Halten eines Abschnitts einer Schleuse, hier lediglich als Verbesserung des Systems des zweiten Aspekts beschrieben wurde, kommt auch ihr eine eigenständige Bedeutung zu. Die gemeinsame erfinderische Idee ist es, die Pumpe vor dem Einführen in das die Pumpe aufzunehmende Lumen ohne Zeitdruck in eine zweite Schleuse einzuführen.

Alle drei Aspekte sind für sich genommen geeignet, ein Abknicken der Welle einer Pumpe zu verhindern.

Im Folgenden wird auf weitere Ausführungsformen der unterschiedlichen Aspekte eingegangen.

In einer Ausführungsform des ersten Aspekts der Erfindung weist das erste Schleusenlumen bereichsweise eine kleinere Innenquerschnittsfläche, vorzugsweise einen kleineren Innendurchmesser, als das zweite Schleusenlumen auf. Dies hat den Vorteil, dass das Einfädeln der Pumpe in die zweite Schleuse vereinfacht vorgenommen werden kann. Als geeignetes Größenverhältnis der Innenquerschnittsfläche des ersten Schleusenlumens zu der des zweiten Schleusenlumens bietet sich ein Verhältnis zwischen 1:1 und 1:1,2 an.

In einer weiteren Ausführungsform des Systems des ersten Aspekts umfasst die erste Schleuse an ihrem proximalen Ende ein erstes Schleusenventil. Mithilfe des Schleusenventils kann die Einführschleuse zunächst in das die Pumpe aufnehmende Lumen eingeführt werden und ein Auslaufen eines Fluids aus dem Lumen verhindert werden.

Dabei ist das Schleusenventil bevorzugt derart gestaltet, dass es einen breiteren Innenquerschnitt als das Schleusenlumen aufweist. Auch eine konisch zulaufende Form des Schleusenventils wird von der Ausführungsform umfasst. Dabei verjüngt sich das Schleusenventil von seinem proximalen Ende zum distalen Ende hin, d. h. zum demjenigen Ende, an welchem es mit dem Schleusenlumen verbunden wird. Alternativ oder in Kombination kann das erste Schleusenlumen derart ausgebildet sein, dass sich dies im Bereich der Verbindung zwischen dem Schleusenlumen und dem Schleusenventil aufweitet.

Die Schleuse ist erfindungsgemäß derart ausgebildet, dass das distale Ende der zweiten Schleuse in das erste Schleusenventil zumindest teilweise einführbar ist und die Kopplung zwischen der ersten und zweiten Schleuse hierüber hergestellt wird.

In einer Ausführungsform des ersten und zweiten Aspekts der Erfindung ist die zweite Schleuse eine über ihre gesamte Länge aufreißbare Aufreißschleuse bzw. eine "Peel-away"-Schleuse. Derartige Schleusen besitzen beispielsweise eine molekulare Struktur, welche ein Aufreißen vom proximalen Ende zum distalen Ende hin erleichtert. Die Aufreißschleuse kann aber auch beispielsweise durch eine Materialverjüngung nach Art einer Sollbruchstelle oder durch einen längs eingelegten Aufreißdraht bzw. -faden aufreißbar ausgeführt sein. Hierdurch kann die zweite Schleuse, nachdem die Pumpe von der zweiten Schleuse in die erste Schleuse überführt wurde, aufgerissen und von der Pumpe, insbesondere vom in ihr verlaufenden Wellenkatheter, abgezogen werden. Die zweite Schleuse stellt in dieser Variante ein Merkmal der Erfindung dar, welches lediglich vor dem Einführen in das die Pumpe aufnehmende Lumen mit der Pumpe verbunden ist.

In einer weiteren Ausführungsform des ersten und zweiten Aspekts der Erfindung umfassen die erste und die zweite Schleuse einen Kunststoff, welcher in einer weiteren Ausführungsform mit einer verstärkenden bzw. versteifenden Struktur versehen sein kann. Kunststoff stellt im medizinischen Bereich eine besonders geeignete Materialwahl dar.

In einer Ausführungsform des dritten Aspekts umfasst die Vorrichtung zum Halten eines Abschnitts der zweiten Schleuse einen Hohlraum, in welchem der Abschnitt der zweiten Schleuse kraftschlüssig gehalten werden kann. Dies kann beispielsweise über eine aufgeraute Oberfläche'des Hohlraums realisiert werden. Eine andere Möglichkeit besteht darin, die Innenquerschnittsfläche des Hohlraums minimal kleiner als die Außenquerschnittsfläche der zweiten Schleuse auszubilden. Dadurch, dass die Vorrichtung einen Hohlraum aufweist, können die den Hohlraum umgebenden Flächen als Haltefläche für einen Bediener des Systems dienen. Auch hierdurch wird eine Beschädigung der Welle vermieden. Als Material bietet sich beispielsweise Metall oder auch ein Kunststoff an, um die Übertragung von Erregern von der Vorrichtung auf die Schleuse zu vermeiden. Im Fall der Verwendung in einem menschlichen oder tierischen Körper sind vorzugsweise biokompatible Materialien zu verwenden.

In einer weiteren Ausführungsform des dritten Aspekts ist die Vorrichtung derart ausgebildet, dass die zweite Schleuse bevorzugt über mindestens die Hälfte ihrer Länge durch die Vorrichtung zum Halten der zweiten Schleuse aufnehmbar ist.

In einer weiteren Ausführungsform ist die Vorrichtung derart ausgebildet, dass der gehaltene Abschnitt der zweiten Schleuse eine Krümmung von weniger als 10°, vorzugsweise weniger als 5°, besonders vorzugsweise weniger als 1° enthält. Durch eine Krümmung der zweiten Schleuse wächst die Gefahr eines Abknickens der Welle. Dies kann durch eine entsprechende Ausbildung der Vorrichtung zum Halten eines Abschnitts der zweiten Schleuse vermieden werden.

Durch eine Krümmung des Aufnahmebereichs der zweiten Schleuse an der Vorrichtung wird die zweite Schleuse axial fixiert ohne das Lumen L2 der zweiten Schleuse einzuschränken. Hierzu soll die 2. Schleuse eine angemessene Biegesteifigkeit besitzen. Alternativ kann auch die 2. Schleuse eine Krümmung besitzen und die Vorrichtung gerade (oder beide gekrümmt) sein.

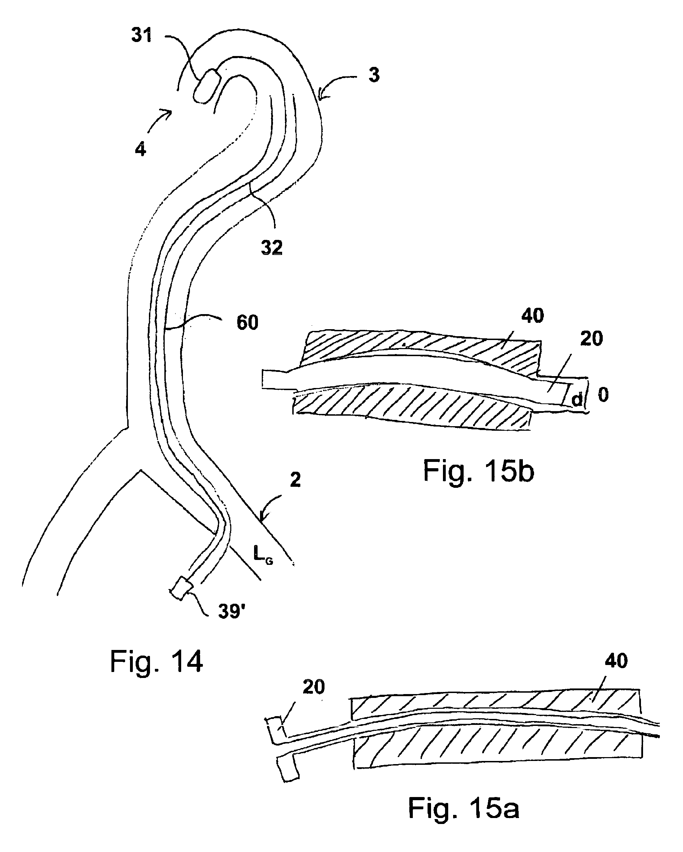

Hierzu sieht ein erfindungsgemäßes System vor, dass die Gradheitsabweichungen der zweiten Schleuse im geklemmten Bereich und der Vorrichtung zum Halten eines Abschnitts der zweiten Schleuse zusammen größer sind als die Differenz des Innendurchmessers der Vorrichtung zum Halten eines Abschnitts der zweiten Schleuse und des Außendurchmessers der zweiten Schleuse, siehe Fign. 15a und 15b.

Das System nach dem ersten Aspekt kann in Verbindung mit einem System nach dem zweiten Aspekt derart verwendet werden, dass zunächst die erste Schleuse in das die Pumpe später aufnehmende Lumen eingeführt wird, derart, dass das proximale Ende der ersten Schleuse außerhalb des aufnehmenden Lumens zugänglich ist. Davor oder danach kann das distale Ende der Pumpe in das Schleusenlumen der zweiten Schleuse bewegt werden, vorzugsweise mithilfe eines Systems nach dem zweiten Aspekt in die zweite Schleuse eingezogen werden.

Hierzu kann die zweite Schleuse in ihrem distalen Bereich zumindest axial versteift sein. Somit kann ein Knicken der Pumpe bzw. der zweiten Schleuse beim Einziehen der Pumpe in die Schleuse verhindert werden.

Anschließend wird das distale Ende der zweiten Schleuse an das proximale Ende der ersten Schleuse gekoppelt und die Pumpe von der zweiten Schleuse in die erste Schleuse überführt.

In einer Ausführungsform wird anschließend das distale Ende der Pumpe durch das distale Ende des ersten Schleusenlumens in das die Pumpe aufnehmende Lumen geführt.

In einem Verfahren zum Ausführen der Pumpe wird die distal des distalen Endes der im Gefäßsystem verbleibenden Schleuse gelegene distale Pumpeneinheit in die Schleuse eingezogen und anschließend durch die Schleuse aus dem aufnehmenden Lumen entfernt. Alternativ kann die Pumpe zusammen mit dem aufnehmenden Lumen entfernt werden.

In einer Ausführungsform des Verfahrens zum Ausführen der Pumpe liegt das distale Ende der während des Arbeitszustands der Pumpeneinheit weniger als 20 cm, vorzugsweise weniger als 10 cm vom Wirkungsort der Pumpeneinheit entfernt. Hierdurch wird der Transport der Pumpeneinheit zum Wirkungsort und von diesem hinweg vereinfacht. Darüberhinaus wird auch das Gefäßsystem hierbei vor Läsionen durch die Pumpe (beim Transport derselben) gestützt.

Die vorliegende Patentanmeldung bezieht sich außerdem auf eine Katheterpumpe, welche in einem komprimierten Zustand zum Einführen in menschliche oder tierische Gefäße einführbar ist und in einem expandierten Zustand in einem Lumen des menschlichen oder tierischen Körpers betreibbar ist, wobei die Katheterpumpe einen Rotor zum Fördern von Fluid, ein den Rotor umgebendes, zumindest teilweise fluiddurchlässiges Gehäuse sowie eine rotornahe Einzugsschleuse aufweist, wobei beim Überführen vom expandierten in den komprimierten Zustand das Gehäuse in die Einzugsschleuse zur zumindest bereichsweisen Durchmesserverengung des Gehäuses einbringbar ist und diese Durchmesserverengung des Gehäuses derart bemessen ist, dass auch der Durchmesser (d.h. der größte Außendurchmesser) des Rotors durch die Durchmesserverengung des Gehäuses verringert wird.

Die im vorangegangenen Absatz beschriebene Erfindung ist für sich genommen für Katheterpumpen eine eigenständige Erfindung, so dass sich der Anmelder vorbehält, hierauf später eine gesonderte Teilanmeldung zu richten.

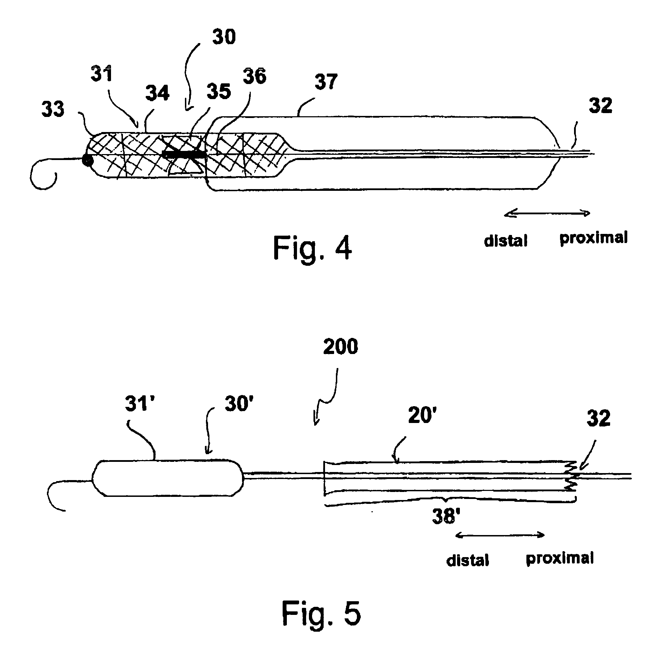

Vorteilhafte Weiterbildungen dieser Katheterpumpe (siehe vorherigen Absatz) sind aus den ursprünglich eingereichten Fign. 1 bis 15b erkennbar, insbesondere aus Fign. 4, 5 oder 6.

Sämtliche hier genannte Ausführungsformen bzw. ursprünglich eingereichte Patentansprüche sollen, sofern dies technisch nicht widersprüchlich ist, als mögliche Weiterbildungen gelten.

Das im ursprünglich eingereichten Patentanspruch 23 genannte Grundprinzip beruht darauf, dass die Einführschleuse den Rotor nicht selbst komprimiert, sondern dass dies mittelbar durch ein den Rotor umgebendes Gehäuse geschieht. Dieses Gehäuse kann (wie in dieser Anmeldung gezeigt) eine Gitterstruktur aufweisen, welche bereichsweise verschlossen ist. Hierdurch ist ein Einsaugen von Fluid in das Gehäuse möglich und eine Förderung zum proximalen Ende des Katheters hin möglich. Der genannte Rotor kann verschiedene Formen aufweisen, beispielsweise kann er verschiedene Rotorblätter haben, wobei diese einzelnen Rotorblätter auch Lamellen aufweisen können. Der Rotor kann auch eine äußere Kunststoffoberfläche haben und beispielsweise als Kunststoffgieß- oder Spritzgussteil ausgeführt sein. Wichtig ist, dass der Rotor auch eine komprimierte Stellung zum Einführen in das Gefäß (bzw. das Gehäuse/die Schleuse) aufweisen kann sowie eine expandierte Stellung, in der ein Förderbetrieb möglich ist und in dem der Rotor durch das umgebende Gehäuse geschützt wird. Hierbei kann ein Spalt zwischen den radialen Rotoraußenkanten sowie dem Gehäuse bestehen. Selbst im Betrieb kann das Gehäuse noch bereichsweise in der rotornahen Einführschleuse angeordnet sein, solange hierdurch eine freie Drehung des Rotors nicht behindert wird.

Die oben genannten Erfindungen eignen sich also insbesondere für selbstentkomprimierbare Pumpen.

Nachfolgend soll die Erfindung anhand einiger Ausführungsbeispiele genauer erläutert werden. Es zeigen:
- Fig. 1: eine schematische Übersicht über ein Gefäßsystem mit einer eingeführten ersten Schleuse;
- Fig. 2: eine Detailansicht eines Ausschnitts der Fig. 1;
- Fig. 3: eine Ausführungsform eines Systems gemäß dem ersten Aspekt der Erfindung;
- Fig. 4: eine Ausführungsform einer Pumpe;
- Fig. 5: eine Ausführungsform eines Systems gemäß dem zweiten Aspekt der Erfindung;
- Fign. 6, 7: das Einziehen einer Pumpe in eine zweite Schleuse;
- Fig. 8, 9: das Überführen einer Pumpe von einer zweiten in eine erste Schleuse;
- Fig. 10: eine Ausführungsform des Systems nach dem zweiten Aspekt mit einer Vorrichtung nach dem dritten Aspekt der Erfindung;
- Fig. 11: einen Querschnitt der Darstellung der Fig. 10;
- Fig. 12: eine Ausführungsform nach dem ersten und dritten Aspekt der Erfindung;
- Fig. 13a und 13b: eine Ausführungsform eines distalen Endes einer Schleuse nach dem zweiten Aspekt der Erfindung;
- Fig. 14: eine Ausführungsform einer langen Schleuse und deren Anordnung während des Arbeitszustands der Pumpeneinheit.
- Fig. 15a und 15b: Skizzen zur Verdeutlichung von Krümmungen.

Wie bereits eingangs erwähnt, zeigt die Fig. 1 eine schematische Darstellung eines menschlichen Gefäßsystems, bei welchem eine als Einführschleuse ausgebildete erste Schleuse 10 unter Anwendung der Seldinger-Technik in die femorale Arterie eingeführt wurde.

In der Fig. 1 ist ferner ein Führungsdraht 12 gezeigt, welcher sich bis in den linken Herzventrikel erstreckt. Der schlauchförmige Abschnitt 11 der ersten Schleuse 10 ist derart in die Arterie eingeführt, dass das proximale Ende der ersten Schleuse 10 außerhalb der femoralen Arterie liegt und somit zum Einführen beispielsweise einer Pumpe genutzt werden kann. Zum einen ist es möglich, die Pumpe auf dem Führungsdraht 12 aufzufädeln, um die Pumpe mithilfe des Führungsdrahtes bis in den linken Herzventrikel zu führen.

Ein erfindungsgemäßes Verfahren ist es, den schlauchförmigen Abschnitt 11 der ersten Schleuse 10 durch den Führungsdraht geführt bis in den linken Herzventrikel zu führen und den Führungsdraht 12 anschließend aus der ersten Schleuse zu entfernen. Eine etwaige Pumpeneinheit wird anschließend durch das erste Schleusenlumen bis in die Nähe oder bis in den linken Ventrikel 4 geführt.

Vorliegend wird die Erfindung lediglich anhand des Einführens einer Pumpe in den linken Herzventrikel zum Unterstützen einer Herzfunktion dargestellt. Für den Fachmann ist jedoch leicht erkennbar, dass die Pumpe auch an andere Stellen im körpereigenen Gefäßsystem angeordnet und eingebracht werden kann. Weiterhin ist für den Fachmann ersichtlich, dass die Erfindung nicht ausschließlich auf das körpereigene Gefäßsystem beschränkt ist, sondern sich zum Einführen einer Pumpe in jegliche Lumina eignet. Dies umfasst beispielsweise Lumina, welche durch Rohre definiert werden, in die eine Pumpeinheit zur Flussförderung eingebracht werden soll.

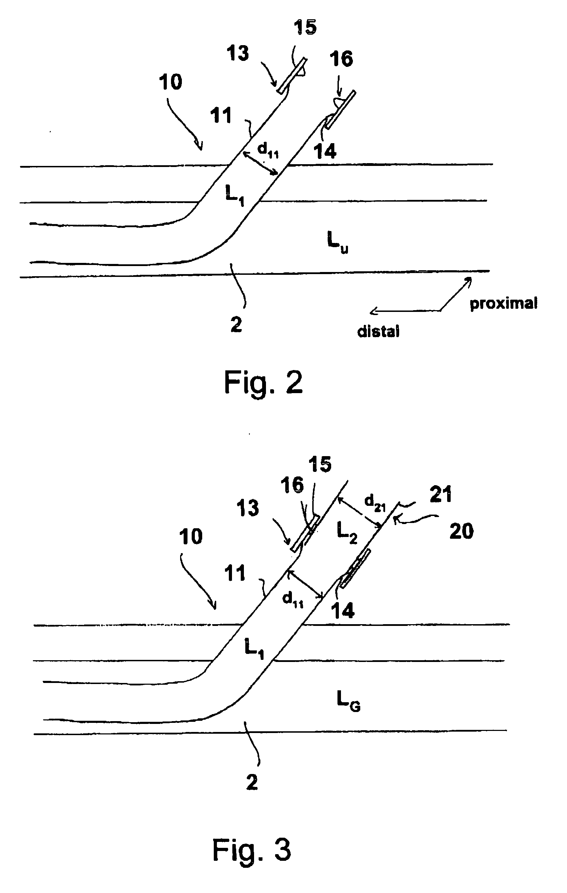

In der Fig. 2 ist der Bereich der Fig. 1 dargestellt, in welchem die erste Schleuse 10 durch das körpereigene Gewebe von außen in das Lumen L_{G} der femoralen Arterie 2 geführt ist. Die erste Schleuse umfasst dabei einen schlauchförmigen Abschnitt 11, welcher proximal mit einem Ventil 13 verbunden ist. Der schlauchförmige Abschnitt 11 definiert ein Lumen L₁, welches einen Innendurchmesser d₁₁ aufweist. Zum proximalen Ende des schlauchförmigen Abschnitts 11 hin weitet sich dieser trompetenartig auf. Die trompetenartige Aufweitung 14 führt dazu, dass der Durchmesser des Ventils 13 größer ist als der Innendurchmesser d₁₁.

Bei dem Ventil 13 handelt es sich um ein im Stand der Technik bekanntes hämostatisches Ventil, welches ein Gehäuse 15 und eine Dichtung 16 umfasst. Das hämostatische Ventil verhindert, dass im Lumen L_{G} befindliches Fluid durch das Lumen L₁ nach außen austreten kann.

In der Darstellung der Fig. 3 ist die erste Schleuse 10 der Fig. 2 mit einer zweiten Schleuse 20 gekoppelt. Von der zweiten Schleuse 20 ist lediglich ein schlauchförmiger Abschnitt 21 gezeigt, welcher ein Lumen L₂ mit einem Innendurchmesser d₂₁ definiert. Das distale, mit dem Ventil 13 gekoppelte Ende der zweiten Schleuse 20 weist dabei einen derartigen Außendurchmesser auf, dass es in das Ventil 13 eingeführt werden kann. Der Innendurchmesser d₂₁ ist jedoch größer als der Innendurchmesser d₁₁.

Eine nicht dargestellte, im Lumen L₂ befindliche Pumpe kann nun durch Drücken vom zweiten Schleusenlumen L₂ in das erste Schleusenlumen L₁ überführt werden. Anschließend wird die Pumpe durch das erste Schleusenlumen L₁ bis an die Stelle im Gefäßsystem transportiert, an welcher die Pumpe ihre Wirkung entfalten soll. Hierbei kann die Pumpe entweder auf einem Führungsdraht geführt werden oder ohne Führungsdraht durch das erste Schleusenlumen eingebracht werden.

Anhand der Fig. 4 wird eine mögliche Ausführung einer Pumpe 30 genauer erläutert. Die Pumpe 30 umfasst eine distale Pumpeneinheit 31 und einen sich an das proximale Ende der distalen Pumpeneinheit 31 anschließenden Wellenkatheter 32. Der Wellenkatheter 32 weist an seinem proximalen, nicht dargestellten Ende eine Kupplung zur Ankopplung des Wellenkatheters 32 an eine Antriebsvorrichtung auf. Die Antriebsvorrichtung versetzt eine im Wellenkatheter 32 verlaufende flexible Welle in Rotation, welche wiederum die distale Pumpeneinheit 31 antreibt.

Die distale Pumpeneinheit umfasst ein Pumpengehäuse 33, welches aus sich kreuzenden Nitinolstreben hergestellt ist. Das Nitinolgehäuse ist zu Teilen mit einer Beschichtung 34 versehen, welche sich distal und proximal eines im Gehäuse 33 angeordneten Rotors 35 erstreckt. Der Rotor ist mit der durch den Wellenkatheter 32 verlaufenden Welle 36 verbunden und wird so in Drehung versetzt. Das Gehäuse und der Rotor sind komprimierbar, d. h., die Pumpe ist eine selbstentkomprimierbare Pumpe. Die Entfaltung der Pumpe vollzieht sich nach dem Herausschieben der distalen Pumpeneinheit aus dem distalen Ende einer Schleuse. Zum Komprimieren der Pumpe wird die distale Pumpeneinheit in das distale Ende eines Schleusenlumens eingezogen. Das Schleusenlumen besitzt dabei einen Innendurchmesser, welcher zumindest größer als der Außendurchmesser des Wellenkatheters ist.

Optional weist die Pumpe einen Abströmschlauch 37 auf, welcher einen proximal des Rotors 35 gelegenen Strömungskanal für das gepumpte Fluid definiert. Am proximalen Ende des Abströmschlauchs 37 befinden sich nicht näher dargestellte Auslassöffnungen.

Selbstverständlich kann die Pumpe auch von einem Pumpbetrieb in einen Saugbetrieb umgeschaltet werden, so dass die Pumpe nicht länger Fluid vom distalen Ende ans proximale Ende führt, sondern umgekehrt.

Eine detailliertere Beschreibung einer weiteren geeigneten Pumpe kann beispielsweise der Druckschrift EP 2 047 872 A1 entnommen werden.

Anhand der Figuren 5 bis 9 soll nun die Funktion des Systems gemäß dem zweiten Aspekt erläutert werden.

In der Fig. 5 ist eine Pumpe 30' dargestellt, welche im Wesentlichen der Pumpe 30 nach der Fig. 4 entspricht. Zur Vereinfachung sind Details der Pumpe nicht gezeigt. Es sind lediglich das bauchige Gehäuse sowie das distal des bauchigen Gehäuses gelegene "Pigtail" dargestellt, welches ein Ansaugen der Herzpumpe an die Herzwand verhindert. Proximal der distalen Pumpeneinheit 31' verläuft der Wellenkatheter 32'. Einen Bereich 38' des Wellenkatheters 32' umschließend ist eine zweite Schleuse 20' angeordnet, welche ein Lumen L₂ umfasst, dessen Innendurchmesser d₂₁ kleiner als der Durchmesser der distalen Pumpeneinheit 31' ist.

Die in der Fig. 5 dargestellte Pumpe 30' ist eine komprimierbare Pumpe, das heißt, die distale Pumpeneinheit 31', welche unter anderem das Pumpengehäuse und den darin befindlichen Rotor umfasst, ist derart ausgebildet, dass sie komprimiert, d. h. in ihrem Durchmesser verringert werden kann. Nachdem ein Qualitätsprüfer oder beispielsweise ein Arzt sich von der korrekten Funktion der Pumpe 30' überzeugen konnte, z. B. durch Betrachtung der Rotationsbewegung der in der distalen Pumpeneinheit 31' befindlichen Rotoreinheit bei einem Testlauf, wird durch Ziehen des Wellenkatheters 32' in proximaler Richtung die distale Pumpeneinheit 31' in das Lumen L₂ der zweiten Schleuse 20' gezogen. Durch das Einziehen der Pumpe in die zweite Schleuse 20' wird ein Verbiegen bzw. eine Beschädigung des Wellenkatheters bzw. der darin verlaufenden Welle vermieden. Die in der Fig. 5 dargestellte Pumpe 30' und die den Bereich 38' des Wellenkatheters 32' umschließende zweite Schleuse 20' bilden ein System 200, welches es ermöglicht, lange vor einer Operation die Funktion der Pumpe 30' zu testen und anschließend durch das Ziehen der distalen Pumpeneinheit 31' in das distale Ende der zweiten Schleuse 20' die Pumpe zu komprimieren und dabei eine Beschädigung der Welle zu vermeiden.

Obgleich das System gemäß dem ersten und zweiten Aspekt sowohl mit aktiv entkomprimierbaren Pumpen als auch mit selbstentkomprimierbaren Pumpen realisierbar ist, eignet es sich insbesondere für selbstentkomprimierbare Pumpen, d.h. Pumpen, deren distale Pumpeneinheit außerhalb der Schleuse selbsstätig wieder die ursprüngliche Größe annimmt.

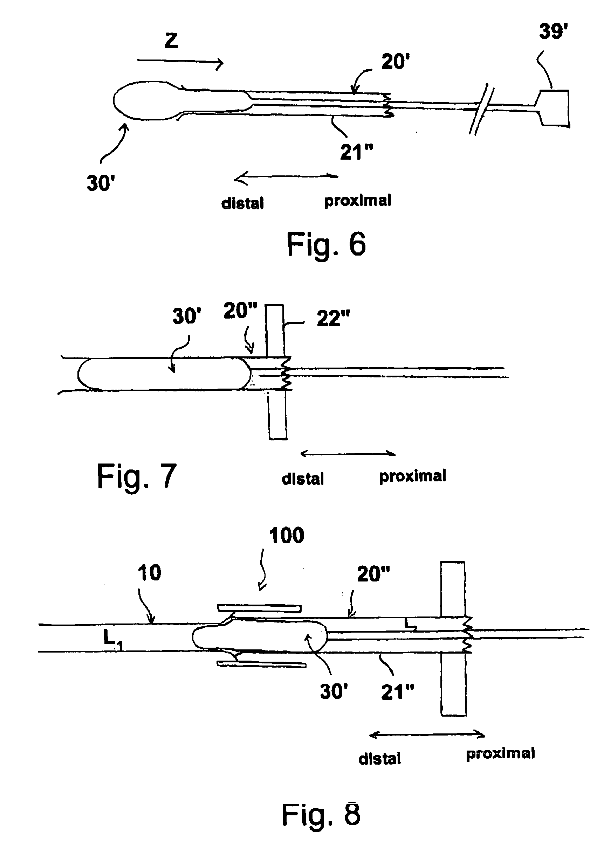

In der Fig. 6 ist ein Zwischenschritt beim Einziehen der distalen Pumpeneinheit 31' in das Lumen L₂ der zweiten Schleuse 20' dargestellt. Es ist erkennbar, dass die distale Pumpeneinheit 31' komprimierbar ist und auf einen geringeren Durchmesser gebracht werden kann, so dass die distale Pumpeneinheit 31' in das Lumen L₂ der zweiten Schleuse 20' aufgenommen werden kann.

Ferner ist in der Fig. 6 eine an den Wellenkatheter 32' anschließende Kupplung 39' dargestellt, welche ein Ankoppeln der in dem Wellenkatheter verlaufenden Welle an eine Antriebseinheit ermöglicht. Da die Kupplung 39' einen oftmals größeren Außendurchmesser als den Innendurchmesser des Lumens L₂ aufweist, wird die zweite Schleuse 20' zumeist vor der Montage der Kupplung 39' vom proximalen Ende des Wellenkatheters 32' her in distaler Richtung aufgesetzt, so dass die Pumpe im System 200, d. h. die Pumpe mit der sich proximal der distalen Pumpeneinheit 31' befindlichen zweiten Schleuse 20' und der vormontierten Kupplung 39', ausgeliefert wird. An der Fig. 6 ist auch eine leichte trompetenförmige Aufweitung des distalen Endes der zweiten Schleuse 20' dargestellt. Die trompetenartige Aufweitung 24' erleichtert das Einziehen der distalen Pumpeneinheit 31' in das Lumen L₂ der zweiten Schleuse 20'.

In der Fig. 7 schließlich befindet sich die distale Pumpeneinheit 31' vollständig im Lumen L₂ der zweiten Schleuse 20". Die zweite Schleuse 20" unterscheidet sich von der Schleuse 20' lediglich durch zwei vormontierte Griffeinheiten 22", welche ein besseres Halten bzw. Entfernen der zweiten Schleuse 20" beim Einziehen der distalen Pumpeneinheit 31' in das Lumen L₂ bzw. ein anschließendes Aufreißen ermöglichen. Vorteilhafterweise wird bei vorhandenem "Pigtail" dieses ebenfalls in das Lumen L₂ eingezogen, so dass sich die distale Pumpeneinheit 31' samt distal der distalen Pumpeneinheit 31' gelegenen Komponenten der Pumpe im Lumen L₂ befindet.

In der Fig. 8 wird erkennbar, wie das System 200 aus Pumpe 30' und zweiter Schleuse 20" in Wirkverbindung mit der ersten Schleuse 10 zu einem System 100 nach dem ersten Aspekt der Erfindung kombiniert wird. Zunächst wird die zweite Schleuse 20" mit ihrem distalen Ende in das Ventil der ersten Schleuse 10 eingeführt. Sobald die distale Spitze der zweiten Schleuse 20" an der trompetenförmigen Aufweitung 14 der ersten Schleuse 10 anliegt, wird durch Schieben der Pumpe in distaler Richtung, wobei das Schieben anhand eines Schiebens des Wellenkatheters 32' erfolgt, die Pumpe von der zweiten Schleuse 20' in die erste Schleuse 10' überführt. Hierbei wird der Durchmesser der distalen Pumpeneinheit 31' weiter auf den Innendurchmesser d₁₁ des Lumens L₁ reduziert.

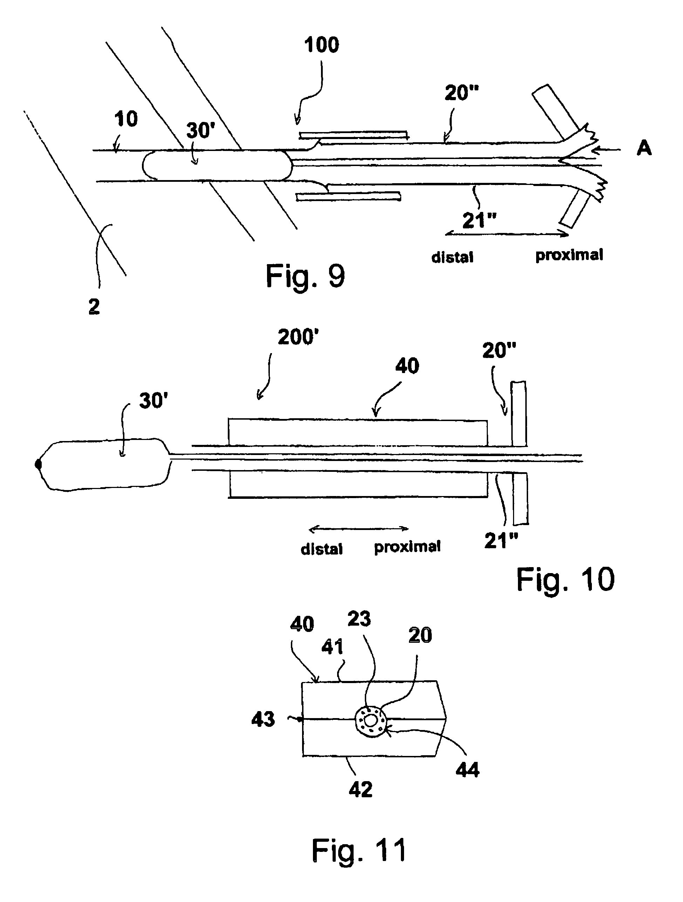

In der Fig. 9 ist der anschließende Schritt dargestellt, bei welchem sich die distale Pumpeneinheit 31' vollständig im Lumen L₁ der ersten Schleuse 10 befindet. Dass sich die distale Pumpeneinheit 31' vollständig im Lumen L₁ der ersten Schleuse 10 befindet, kann beispielsweise anhand einer farblichen Markierung 50, welche auf der Außenseite des Wellenkatheters 32' aufgebracht ist, kenntlich gemacht werden.

Anschließend wird die zweite Schleuse 20", welche als eine "Peel-away"-Schleuse ausgebildet ist, vom Wellenkatheter 32' entfernt, indem die Peel-away-Schleuse vom proximalen zum distalen Ende hin aufgerissen und vom Wellenkatheter 32' abgezogen wird. Das gerichtete Aufreißen vom proximalen zum distalen Ende hin kann durch Einkerbungen A unterstützt werden, beruht jedoch vorwiegend auf der Ausrichtung der Molekülketten des verwendeten Kunststoffs von der proximalen in die distale Richtung.

Nachdem die Peel-away-Schleuse entfernt wurde, wird die Pumpe 30' weiter innerhalb des Lumens L₁ der ersten Schleuse 10 bis zum gewünschten Ort geführt.

Eine Versteifung der zweiten Schleuse 20" ist insbesondere beim Einziehen der distalen Pumpeneinheit 31' in das distale Ende des zweiten Schleusenlumens L₂ nicht notwendig, da die Gefahr eines Abknickens der Welle bei einer Ziehbewegung stark reduziert ist.

Beim Überführen der Pumpe von der zweiten Schleuse in die erste Schleuse, wie anhand der Figuren 7 bis 9 dargestellt, kann die zweite Schleuse eine sie verstärkende Struktur in Form eines eingebrachten Drahtes umfassen, oder der schlauchförmige Abschnitt 21" der Schleuse 20" wird nicht aus einem flexiblen Kunststoff, sondern aus einem formfesten Kunststoff oder Metall hergestellt.

Eine weitere Möglichkeit zum Einziehen der Pumpe in die zweite Schleuse besteht darin, beim Vorschieben in der Pumpe 30' in distaler Richtung, d. h. insbesondere beim Überführen der Pumpe 30' von der zweiten Schleuse in die erste Schleuse, die zweite Schleuse 20" durch einer Vorrichtung 40 zum Halten der zweiten Schleuse zu halten.

Die Vorrichtung zum Halten eines Abschnitts der zweiten Schleuse 20" ist im Querschnitt in der Fig. 11 dargestellt. Die Vorrichtung 40 weist eine obere und eine untere Hälfte 41 bzw. 42 auf, welche über ein Scharnier 43 miteinander verbunden sind. In der ersten bzw. zweiten Hälfte 41 bzw. 42 befindet sich jeweils eine Ausnehmung, wobei die Ausnehmungen im dargestellten, geschlossenen Zustand der Vorrichtung 40 einen Hohlraum 44 definieren. Der als Lumen ausgebildete Hohlraum 44 ist weiterhin derart ausgebildet, dass eine zweite Schleuse 20 in dem Hohlraum kraftschlüssig gehalten werden kann. Dies kann entweder über entsprechend gewählte Innendurchmesser des Hohlraums 44 bzw. Außendurchmesser der zweiten Schleuse 20 oder raue Oberflächen im Bereich des Hohlraums 44 erreicht werden. Auch Noppen im Bereich des Hohlraums 44 können einen Kraftschluss herstellen.

Wie anhand der Fig. 10 erkennbar ist, verläuft der Hohlraum 44 ohne Krümmung und erlaubt somit kein Verbiegen der in dem Wellenkatheter 32' verlaufenden Welle, wenn diese mittels Schubkraft in distaler Richtung vorgeschoben wird. Zudem ermöglicht die Vorrichtung 40 eine verbesserte Griffigkeit für einen Bediener des Systems 200' zum Einführen der Pumpe.

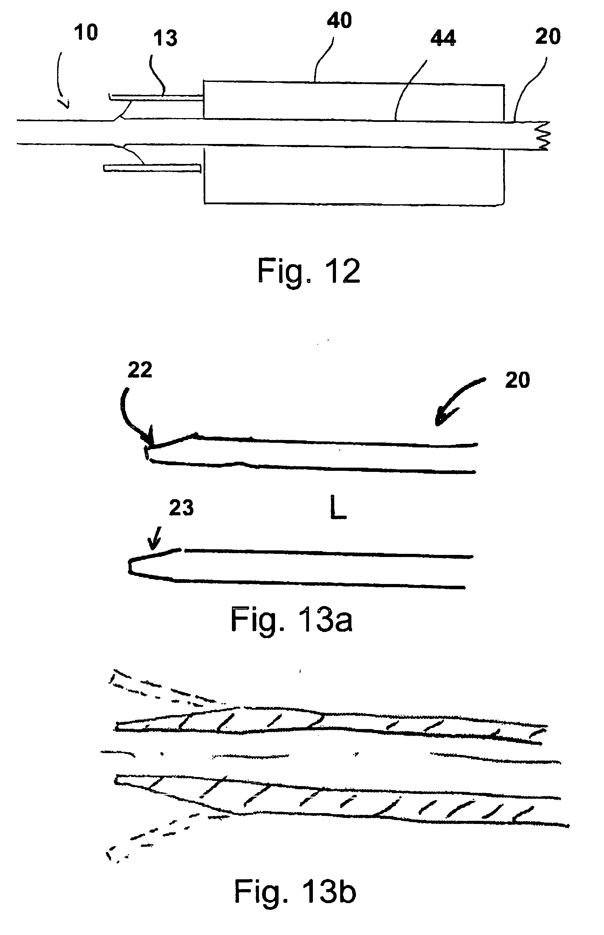

In der Fig. 12 ist die Vorrichtung 40 mit dem Hohlraum 44 dargestellt, in welcher eine zweite Schleuse 20 gehalten ist. Dabei liegt das distale Ende der Vorrichtung 40 auf dem proximalen Ende des Ventils 13 der ersten Schleuse 10 auf. Bei einer derartigen Ankopplung zwischen der ersten Schleuse 10 und der zweiten Schleuse 20 kann ein vereinfachtes Einführen einer Pumpe von der zweiten in die erste Schleuse erreicht werden.

In der Fig. 13a ist ein distales Ende 22 einer Ausführungsform der Schleuse 20 nach dem zweiten Aspekt der Erfindung dargestellt. Das distale Ende 22 weist eine innen- und außenseitige Verjüngung des das Lumen L umschließenden Randes 23 auf, wodurch ein verbessertes Einführen der Schleuse in das körpereigene Lumen oder eine erste Schleuse gemäß dem ersten Aspekt der Erfindung möglich ist. Ebenso kann das distale Ende 22 das Ende einer zweiten Schleuse nach dem ersten Aspekt der Erfindung sein. Ein etwaiges proximales Ende kann formgleich ausgebildet sein.

Fig. 13b zeigt eine weitere Variante; ein zusätzliches Merkmal ist hierbei, dass das distale Ende derart flexibel bzw. elastisch ausgebildet ist, dass es sich eim Einziehen des Pumpenkopfes geringfügig aufweitet und dadurch das Einziehen erleichtert. Nach vollständigem Einziehen des Pumpenkopfes nimmt das distale Ende wieder weitgehend die ursprüngliche Form ein. Beim Einführen des distalen Endes der zweiten Schleuse in die erste Schleuse kann sich das distale Ende der zweiten Schleuse auch durch elastische/flexible Verjüngung an das proximale Ende der ersten Schleuse anpassen und dadurch das Ankoppeln erleichtern.

Die in den vorliegenden Unterlagen dargestellten Systeme gemäß dem ersten, zweiten und dritten Aspekt ermöglichen somit eine vereinfachte Handhabung und ein verbessertes Einführen von Pumpen in ein Lumen.

Anschließend soll noch eine mögliche Variante eines Verfahrens zum Einführen einer Pumpe in einen linken Herzventrikel geschildert werden. Als vorbereitende Maßnahme wird die Pumpe zunächst mit steriler physiologischer Kochsalzlösung befüllt und somit komplett entlüftet. Anschließend wird die proximal der distalen Pumpeneinheit gelegene Peel-away-Schleuse bis zu einem eventuell vorhandenen Abströmschlauch vorgeschoben. Die Peel-away-Schleuse besitzt einen Durchmesser von beispielsweise 10 Fr. Nachdem die Peel-away-Schleuse bis zum Abströmschlauch vorgeschoben wurde, wird die Peel-away-Schleuse von der Vorrichtung zum Halten der zweiten Schleuse umfasst. Anschließend wird die distale Pumpeneinheit, gegebenenfalls unter leichter Drehbewegung, in die Peel-away-Schleuse eingezogen, indem am Wellenkatheter eine Zugbewegung in proximaler Richtung ausgeübt wird. Die Pumpe wird so weit in die zweite Schleuse verschoben, dass ein eventuell vorhandenes Pigtail ebenfalls in der Peel-away-Schleuse geborgen ist.

Durch diese Schritte ist es möglich, die Funktionsfähigkeit der Pumpe bereits vor einem operativen Eingriff zu überprüfen und die Pumpe erst anschließend in eine Schleuse einzuführen, ohne unter Zeitdruck agieren zu müssen. Zum Beispiel erst anschließend wird die Punktur des Gefäßsystems zum Einführen der ersten Schleuse durchgeführt. Zur Zeiteinsparung ist es auf diese Weise aber auch möglich, dass ein Assistent die Pumpe vorbereitet während der Anwender bereits parallel die Punktion durchführt.

Nachdem beispielsweise eine 9Fr-Einführschleuse bis in den linken Herzventrikel eingeführt wurde, wird ein eventuell vorhandener Dilatator aus der Einführschleuse gezogen und aus dieser entfernt.

Anschließend wird die in der Peel-away-Schleuse gehaltene Pumpe, welche wieder von der Vorrichtung zum Halten der zweiten Schleuse umfasst wird, wobei beispielsweise ein 2 cm langer Abschnitt des distalen Endes der Peel-away-Schleuse am distalen Ende der Vorrichtung heraussteht, mit dem Hämostaseventil der Einführschleuse gekoppelt, bis die Spitze der Peel-away-Schleuse innerhalb der Einführschleuse anschlägt. Anschließend wird die Pumpe durch Schieben des Wellenkatheters von der Peel-away-Schleuse in die Einführschleuse überführt. Sobald die distale Pumpeneinheit komplett in die Einführschleuse überführt wurde, wie dies beispielsweise anhand einer optischen Markierung auf dem Wellenkatheterschaft überprüfbar ist, kann die Vorrichtung zum Halten der zweiten Schleuse von der zweiten Schleuse entfernt werden und die Peel-away Schleuse aufgerissen und vom Wellenkatheter abgezogen werden. Anschließend wird die Pumpe innerhalb der Einführschleuse bis in den linken Herzventrikel vorgeschoben. Die Einführschleuse wird anschließend aus dem linken Herzventrikel bis zum Beginn der absteigenden Aorta zurückgezogen.

Die Positionierung der distalen Pumpeneinheit im linken Herzventrikel kann beispielsweise durch Röntgendurchleuchtung kontrolliert werden. Hierzu befindet sich eine röntgensichtbare Markierung am Pumpengehäuse, oder das Pumpengehäuse selbst ist röntgensichtbar. Ebenso sollte der Auslassbereich, d. h. die Ausströmöffnungen eines Abströmschlauches, im Bereich der aufsteigenden Aorta liegen. Auch dies kann mit einer röntgensichtbaren Markierung überprüft werden. Eine eventuell vorhandene Pigtail-Katheterspitze sollte an der Spitze des linken Herzventrikels anstoßen.

Um die Pumpe aus dem Herzventrikel zu entfernen, wird diese mittels am Wellenkatheter aufgebrachter Zugkraft in die Einführschleuse zurückgezogen und komprimiert aus dem arteriellen Gefäßsystem entfernt. Anschließend werden die Einführschleuse und weitere verbliebene Komponenten aus dem Gefäßsystem entfernt.

Ein weiterer Aspekt der Erfindung ist die Verwendung einer langen Schleuse bei der Implantation und Explantation der Pumpe. Die lange Schleuse dient nicht nur, wie im Stand der Technik üblich, zum Einführen der Pumpe in ein körpereigenes Lumen, sondern zur Führung der Pumpe durch das Schleusenlumen in die Nähe des Wirkungsorts. Hierbei ist es vorteilhaft, wenn im medizinischen Bereich die Schleuse eine Länge zwischen 40 und 120 cm besitzt. Die Länge wird zumeist durch den späteren Wirkungsort der Pumpe und die Physis des Patienten bestimmt. Die lange Schleuse kann zum einen durch die erste Schleuse des Systems gemäß dem ersten Aspekt der Erfindung, zum anderen durch die Schleuse eines Systems gemäß dem zweiten Aspekt der Erfindung gegeben sein. Eine schematische Darstellung einer langen Schleuse und der distalen Pumpeneinheit im Arbeitszustand ist der Fig. 14 entnehmbar.

Die lange Schleuse 60, welche durch eine erste oder zweite Schleuse des Systems gemäß dem ersten Aspekt der Erfindung oder eine Schleuse des Systems gemäß dem zweiten Aspekt der Erfindung gebildet ist, endet im Aortenbogen 3, wobei der Wellenkatheter 32 der Pumpe 30 durch das Lumen der langen Schleuse verläuft und am distalen Ende des Schleusenlumens austritt. Die distale Pumpeneinheit 31 befindet sich im Arbeitszustand im Ventrikel 4.

Am proximalen Ende der langen Schleuse befindet sich die Kupplung 39', welche der Ankopplung der im Wellenkatheter verlaufenden Welle an eine Antriebseinheit dient.

Die lange Schleuse weist eine derartige Länge auf, dass eine Platzierung der distalen Pumpeneinheit in der Nähe des Wirkungsorts derselbigen möglich ist.

Der langen Schleuse kommen im Wesentlichen zwei Funktionen zu. Zum einen ermöglicht die lange Schleuse das Führen der Pumpe zum Wirkungsort. Da die Pumpe im Schleusenlumen der langen Schleuse komprimiert ist, ist das Befördern der Pumpe zum Einsatzort gegenüber einer alleinigen Führung durch einen Führungsdraht vereinfacht. Außerdem dient dies dem Vermeiden von Komplikationen oder Verletzen der inneren Gefäßwände. Dies ist insbesondere bei sich selbstentfaltenden Pumpen, d. h. Pumpen, welche über keine aktiv ansteuerbaren Komponenten zur Entfaltung der distalen Pumpeneinheit verfügen, eine erhebliche Vereinfachung gegenüber der Führung der Pumpeneinheit mittels eines Führungsdrahts. Zum anderen ermöglicht die lange Schleuse eine vereinfachte Explantation der Pumpe. Die Pumpeneinheit wird nach Beendigung der Förderleistung durch eine Zugbewegung des Wellenkatheters in das distale Ende der langen Schleuse gezogen, dadurch komprimiert und anschließend entweder durch das Schleusenlumen der langen Schleuse herausgezogen oder zusammen mit der langen Schleuse aus dem körpereigenen Lumen gezogen.

Wird die Pumpe zusammen mit der langen Schleuse aus einem körpereigenen Lumen herausgezogen, wird die Blutung der femoralen Arterie mit einem Druckverband gestillt. Alternativ kann die Pumpe aus dem Schleusenlumen der langen Schleuse herausgezogen werden. Dann kann durch das Lumen der Schleuse ein weiterer Führungsdraht platziert werden, über den dann nach Entfernung der Schleuse eine Vorrichtung zum Schließen der Punktur geführt werden kann. Hierdurch ist eine verbesserte Blutungsstillung erreichbar.

Wird die erste Schleuse des Systems gemäß dem ersten Aspekt der Erfindung als lange Schleuse ausgebildet, kann ein System gemäß dem zweiten Aspekt in das proximale Ende der langen Schleuse eingeführt werden. Anschließend wird die zweite Schleuse gegebenenfalls vom Wellenkatheter abgezogen, und die Pumpe wird durch das Schleusenlumen der langen Schleuse zum Wirkungsort der Pumpe geführt.

Alternativ hierzu kann die lange Schleuse auch durch eine Schleuse im Sinne des Systems des zweiten Aspekts der Erfindung gebildet sein. Auf eine erste Schleuse im Sinne des ersten Aspekts kann dann verzichtet werden. In dieser Variante wird die distale Pumpeneinheit der Pumpe zunächst in das distale Ende des Schleusenlumens der Schleuse gemäß dem zweiten Aspekt der Erfindung eingezogen. Anschließend wird die Schleuse im Sinne des zweiten Aspekts beispielsweise mit Hilfe der Seldinger-Technik in das körpereigene Lumen überführt. Die Schleuse wird dann bis in die Nähe des Wirkungsorts der Pumpe geschoben. Dabei erfolgt das Schieben vorzugsweise an der Außenseite der Schleuse. Um diese Methode anwenden zu können, wird die Pumpe über einen Führungsdraht geführt. Dieser kann z.B. durch ein Führungsdrahtlumen der Katheterspitze oder ein zusätzliches Lumen der Schleuse geführt werden.

Das Befördern der Pumpe zum Wirkungsort ist auf unterschiedliche Art und Weise möglich. Nachdem die Schleuse mit ihrem distalen Ende in einem körpereigenen Lumen liegt, kann die Pumpe durch Schieben des Wellenkatheters aus der Schleuse und außerhalb dieser zum Wirkungsort geführt werden. Hierbei ist darauf zu achten, dass die Pumpeneinheit aufgrund der fehlenden Kompressionskraft des Schleusenlumens im entfalteten Zustand durch das körpereigene Gefäß geschoben werden muss. Bei dieser Variante ist keine lange Schleuse notwendig.

Obgleich in der vorhergehend beschriebenen Variante eine mögliche Alternative des Führens der Pumpeneinheit zum Wirkungsort liegt, ist es bevorzugt, die Pumpe im gefalteten oder komprimierten Zustand innerhalb des Schleusenlumens in die Nähe des Wirkungsorts zu führen. Zur Führung der Schleuse innerhalb des Gefäßsystems kann zum einen eine Rapid-Exchange-Technik verwendet werden, wobei ein Führungsdraht beispielsweise an einer gegebenenfalls vorhandenen Katheterspitze eingekoppelt wird und die Schleuse am Führungsdraht entlang geschoben wird. Alternativ kann die Schleuse ein weiteres Schleusenlumen zur Aufnahme eines Führungsdrahtes umfassen, wobei die Schleuse an den Führungsdraht nach vorne gedrückt wird.

Nachdem die distale Pumpeneinheit aus dem distalen Ende des Schleusenlumens geschoben wurde, wird die Pumpe nur um eine geringe Distanz nach vorne verschoben. Die geringe Distanz beträgt im Falle des menschlichen Herzens als Wirkungsort der Pumpe weniger als 20 cm. Im Verhältnis zur Gesamtlänge der langen Schleuse wird eine geringe Distanz durch ein Verhältnis der Gesamtlänge zur geringen Distanz von 3 zu 1 oder mehr definiert.

Die Schleuse kann über den Zeitraum der Behandlung hinweg im körpereigenen Gefäßsystem - den Wellenkatheter umschließend - verbleiben. In diesem Falle kann die Pumpe zur Explantation in das distale Ende der Schleuse gezogen werden und anschließend, wie in einem der vorhergehenden Abschnitte beschrieben, aus dem körpereigenen Gefäßsystem entfernt werden.

Alternativ kann, falls es sich bei der langen Schleuse um eine Aufreißschleuse handelt, diese nach der Führung der Pumpe zum Wirkungsort abgezogen und entfernt werden. Eine Explantation der Pumpe ist dann nicht länger mittels der Schleuse möglich. Es ist ein zusätzliches Werkzeug erforderlich, welches die Explantation der Pumpe ermöglicht und einen eigenständig schutzfähigen Erfindungsgegenstand bildet.

Das zusätzliche Werkzeug wird erst zum Zeitpunkt der Explantation in das Gefäßsystem eingeführt. Das zusätzliche Werkzeug weist ein Lumen zum Einziehen der distalen Pumpeneinheit auf. Beim Einführen des zusätzlichen Werkzeugs ist darauf zu achten, dass keine unsterilen Bereiche des Wellenkatheters überquert werden. Durch das Queren von unsterilen Bereichen können Keime in das Gefäßsystem eingetragen werden. Um ein keimfreies Einführen des zusätzlichen Werkzeugs zu realisieren, bietet es sich an, einen Abschnitt des im Gefäßsystem liegenden Wellenkatheters aus dem Lumen zu ziehen, da der ehemals im Gefäß liegende Abschnitt üblicherweise keine Fremdkeime aufweist. Der unsterile, während des Pumpeinsatzes außerhalb des Patienten liegende Bereich des Wellenkatheters, welcher sich bis zur Antriebskupplung erstreckt, wird beispielsweise durch Abschneiden entfernt. Auch kann der Wellenkatheter eine geeignete Sollbruchstelle aufweisen. In diesem Fall kann das zusätzliche Werkzeug rohr- bzw. schlauchförmig ausgebildet sein und direkt über das abgetrennte Katheterende geführt werden. Ein eventueller Griff würde die Handhabung erleichtern.

Das Lumen des zusätzlichen Werkzeugs weist einen Innendurchmesser auf, der im Wesentlichen dem Außendurchmesser des zu explantierenden Katheters bzw. dem Außendurchmesser des wieder komprimierten Pumpenkopfes entspricht. Somit ist beim Abtrennen der unsterilen Bereiche des Wellenkatheters darauf zu achten, dass distal des distalen Endes des zusätzlichen Werkzeugs nur Komponenten der Pumpe verbleiben, welche in ein derartig beschaffenes Lumen einziehbar sind.

Alternativ kann das zusätzliche Werkzeug in Form eines längs teilbaren Rohres ausgebildet sein. In diesem Fall wäre ein Abtrennen des unsterilen Katheterbereiches nicht notwendig, da das Werkzeug auch direkt auf dem herausgezogenen Bereich aufgesetzt werden kann.

In den vorhergehenden Beispielen wurde das System gemäß dem zweiten Aspekt der Erfindung derart beschrieben, dass bei der Auslieferung des Systems die Schleuse proximal der distalen Pumpeneinheit liegt und die distale Pumpeneinheit erst durch den Benutzer der Pumpe in das distale Ende des Schleusenlumens der Schleuse gezogen wird. Dies hat den Vorteil, dass der Benutzer die Funktionstüchtigkeit der Pumpe vorab testen kann. Alternativ kann das System auch bereits mit eingezogener distaler Pumpeneinheit ausgeliefert werden. Der Testlauf der Pumpe findet dann werkseitig statt.

Ist die Pumpe eine selbstentkomprimierbare Pumpe, kann die zweite Schleuse des Systems gemäß dem ersten Aspekt der Erfindung, welche unter anderem der Komprimierung der distalen Pumpeneinheit dient, in einer Variante des Systems gemäß dem ersten Aspekt der Erfindung durch ein Crimp-Werkzeug ersetzt werden.

Das Crimp-Werkzeug weist an seinem distalen Ende, welches mit dem proximalen Ende der ersten Schleuse gekoppelt wird, beispielsweise eine Blende auf, welche eine Komprimierung der distalen Pumpeneinheit der Pumpe bewirkt, wenn die distale Pumpeneinheit in die erste Schleuse überführt werden soll. Die Blende kann Teil einer trichterförmigen Verjüngung sein, wobei die Blende am distalen Ende der Verjüngung liegt.

Wird ein Crimp-Werkzeug verwendet, wird die distale Pumpeneinheit durch Schieben in die erste Schleuse des Systems gemäß dem ersten Aspekt der Erfindung überführt.

Zusammenfassend bilden die Systeme nach dem ersten, zweiten und dritten Aspekt eigenständige Erfindungen. Weitere Erfindungsvarianten werden durch ein System gemäß dem zweiten Aspekt mit einer langen Schleuse, ein System nach dem ersten Aspekt mit einer langen ersten Schleuse, ein System nach dem ersten Aspekt mit einer langen zweiten Schleuse, ein System nach dem ersten Aspekt mit einem Crimp-Werkzeug, gegebenenfalls anstatt einer zweiten Schleuse, gegeben. Die entsprechenden Ausführungsformen des Systems nach dem ersten, zweiten und dritten Aspekt sind auf die weiteren Erfindungsvarianten ebenfalls anwendbar.

Die vorgestellten Behandlungsmethoden werden ebenfalls von der Erfindung umfasst. Dies betrifft insbesondere die Implantation und/oder Explantation der Pumpe mittels einer langen Schleuse. Auch die weiteren im vorhergehenden Absatz aufgeführten Erfindungsvarianten eignen sich zur Implantation und/oder Explantation wie in der vorliegenden Anmeldung beschrieben.

## Patentansprüche

1. System (100) zum Einführen einer Pumpe in ein Lumen, eine erste Schleuse (10) und eine in die erste Schleuse einzuführende Pumpe (30; 30') umfassend, wobei die erste Schleuse ein erstes, sich von einem distalen Ende zu einem proximalen Ende hin erstreckendes Schleusenlumen (L₁) zur Führung der Pumpe aufweist,
**dadurch gekennzeichnet, dass** eine zweite Schleuse (20; 20'; 20") mit einem zweiten, sich von einem distalen Ende zu einem proximalen Ende hin erstreckenden Schleusenlumen (L₂) vorhanden ist, in welchem die Pumpe führbar gehalten ist, wobei die zweite Schleuse mit der ersten Schleuse zur Überführung der Pumpe in das erste Schleusenlumen koppelbar ist.

2. System nach Anspruch 1, **dadurch gekennzeichnet, dass** das erste Schleusenlumen (L₁) bereichsweise eine kleinere, gleich große oder eine größere Innenquerschnittsfläche, vorzugsweise einen kleineren, gleich großen oder einen größeren Innendurchmesser (d₁), als das zweite Schleusenlumen (L₂) aufweist.

3. System nach einem der vorhergehenden Ansprüche, **dadurch gekennzeichnet, dass** die erste Schleuse (10) an ihrem proximalen Ende ein erstes Schleusenventil (11) aufweist, wobei das erste Schleusenlumen sich vorzugsweise zu seinem proximalen Ende hin aufweitet und derart ausgebildet ist, dass das distale Ende der zweiten Schleuse (20; 20'; 20") in das erste Schleusenventil (13) einführbar ist.

4. System nach einem der vorhergehenden Ansprüche, **dadurch gekennzeichnet, dass** die zweite Schleuse (20; 20'; 20") eine aufreißbare Aufreißschleuse (20'; 20") ist.

5. System nach einem der vorhergehenden Ansprüche, **dadurch gekennzeichnet, dass** die erste und/oder zweite Schleuse einen Kunststoff aufweist.

6. System nach einem der vorhergehenden Ansprüche, **dadurch gekennzeichnet, dass** ein Führungsdraht (12) umfasst ist, dessen Querschnittsfläche derart gewählt ist, dass der Führungsdraht (12) durch das erste Schleusenlumen (L₁) führbar ist.

7. System nach einem der vorhergehenden Ansprüche, **dadurch gekennzeichnet, dass** ferner eine Vorrichtung (40) zum Halten eines Abschnitts der zweiten Schleuse (20; 20'; 20") vorhanden ist.

8. System nach Anspruch 7, **dadurch gekennzeichnet, dass** die Vorrichtung (40) einen eine Außenseite der zweiten Schleuse entlang des Abschnitts umschließenden Hohlraum (44) aufweist, in welchem die zweite Schleuse kraftschlüssig haltbar ist.

9. System nach einem der Ansprüche 7 oder 8,
**dadurch gekennzeichnet, dass** die zweite Schleuse (20; 20'; 20") über mindestens die Hälfte ihrer Länge durch die Vorrichtung (40) aufnehmbar ist.

10. System nach einem der Ansprüche 7 bis 9, **dadurch gekennzeichnet, dass** die Vorrichtung (40) den gehaltenen Abschnitt der zweiten Schleuse mit einer Krümmung von weniger als 10°, vorzugsweise weniger als 5°, hält.

11. System nach einem der vorhergehenden Ansprüche, **dadurch gekennzeichnet, dass** die erste Schleuse oder die zweite Schleuse eine lange Schleuse ist.

12. System (200; 200') mit einer Pumpe (30; 30'), welche eine distale Pumpeneinheit (31) und einen proximal der Pumpeneinheit verlaufenden Wellenkatheter (32) umfasst,
**dadurch gekennzeichnet, dass** eine proximal der distalen Pumpeneinheit angeordnete Schleuse (20; 20', 20") mit einem Schleusenlumen (L₂) vorhanden ist, wobei das Schleusenlumen einen Abschnitt (38) des Wellenkatheters umschließt und derart ausgebildet ist, dass die Schleuse entlang des Wellenkatheters in Richtung der distalen Pumpeneinheit (31) verschiebbar ist und die distale Pumpeneinheit in das Schleusenlumen (L₂) einführbar ist.

13. System nach Anspruch 12, **dadurch gekennzeichnet, dass** die Schleuse aufreißbare Aufreißschleuse (20'; 20") ist.

14. System nach einem der Ansprüche 12 oder 13,
**dadurch gekennzeichnet, dass** die Schleuse (20; 20'; 20") zumindest in ihrem distalen Bereich versteift ist.

15. System nach einem der Ansprüche 12 bis 14,
**dadurch gekennzeichnet, dass** die Schleuse eine lange Schleuse (60) ist.

16. System nach einem der vorhergehenden Ansprüche, **dadurch gekennzeichnet, dass** die Pumpe eine selbstexpandierbare Pumpe ist.

17. System nach einem der vorhergehenden Ansprüche, **dadurch gekennzeichnet, dass** die Gradheitsabweichungen der zweiten Schleuse im geklemmten Bereich und der Vorrichtung zum Halten eines Abschnitts der zweiten Schleuse zusammen größer sind als die Differenz des Innendurchmessers der Vorrichtung zum Halten eines Abschnitts der zweiten Schleuse und des Außendurchmessers der zweiten Schleuse.

18. Verfahren zum Einführen einer Pumpe in ein Lumen mittels eines Systems (100) zum Einführen einer Pumpe in ein Lumen, eine erste Schleuse (10) und eine in die erste Schleuse einzuführende Pumpe (30; 30') umfassend, wobei die erste Schleuse ein erstes, sich von einem distalen Ende zu einem proximalen Ende hin erstreckendes Schleusenlumen (L₁) zur Führung der Pumpe aufweist, wobei eine zweite Schleuse (20; 20'; 20") mit einem zweiten, sich von einem distalen Ende zu einem proximalen Ende hin erstreckenden Schleusenlumen (L₂) vorhanden ist, in welchem die Pumpe führbar gehalten ist, wobei die zweite Schleuse mit der ersten Schleuse zur Überführung der Pumpe in das erste Schleusenlumen koppelbar ist nach einem der vorhergehenden Ansprüche, **dadurch gekennzeichnet, dass**
a) die erste Schleuse (10) in das die Pumpe (30; 30') später aufnehmende Lumen (L_{G}) eingeführt wird, wobei das proximale Ende der ersten Schleuse außerhalb des aufnehmenden Lumens zugänglich ist;
b) das distale Ende der Pumpe in das Schleusenlumen (L₂) der zweiten Schleuse (20; 20'; 20") bewegt wird, vorzugsweise eingezogen wird;
c) das distale Ende der zweiten Schleuse (20; 20'; 20") an das proximale Ende der ersten Schleuse (10) gekoppelt wird und die Pumpe (30; 30') von der zweiten Schleuse in die erste Schleuse überführt wird.

19. Verfahren nach Anspruch 18, **dadurch gekennzeichnet, dass** das distale Ende der Pumpe (30; 30') durch das distale Ende des ersten Lumens (L₁) oder des zweiten Lumens (L₂) in das aufnehmende Lumen (L_{G}) geführt wird.

20. Verfahren zum Einführen einer Pumpe in ein Lumen mittels eines Systems (200; 200') mit einer Pumpe (30; 30'), welche eine distale Pumpeneinheit (31) und einen proximal der Pumpeneinheit verlaufenden Wellenkatheter (32) umfasst,
**dadurch gekennzeichnet, dass** eine proximal der distalen Pumpeneinheit angeordnete Schleuse (20; 20', 20") mit einem Schleusenlumen (L₂) vorhanden ist und das Schleusenlumen einen Abschnitt (38) des Wellenkatheters umschließt und derart ausgebildet ist, dass die Schleuse entlang des Wellenkatheters in Richtung der distalen Pumpeneinheit (31) verschiebbar ist und die distale Pumpeneinheit in das Schleusenlumen (L₂) einführbar ist,
wobei die zweite Schleuse direkt oder über eine weitere Schleuse durch eine Punktur des Lumens in das Lumen eingeführt wird.

21. Verfahren zum Ausführen einer Pumpe aus einem Lumen mittels einer Schleuse, wobei die Pumpe eine distale Pumpeneinheit und einen sich proximal der Pumpeneinheit erstreckenden Wellenkatheter umfasst und die Schleuse ein sich von einem distalen Ende zu einem proximalen Ende hin erstreckendes Schleusenlumen (L₁, L₂) zur Führung der Pumpe aufweist und das Schleusenlumen den Wellenkatheter teilweise umschließt,
**dadurch gekennzeichnet, dass** die distale Pumpeneinheit durch das distale Ende des Schleusenlumens in das Schleusenlumen gezogen wird und anschließend durch das proximale Ende des Schleusenlumens entfernt wird oder mit dem Schleusenlumen aus dem Lumen entfernt wird.

22. Verfahren nach Anspruch 21, **dadurch gekennzeichnet, dass** die Schleuse eine erste Schleuse oder eine Aufreißschleuse ist.

23. Katheterpumpe, welche in einem komprimierten Zustand in menschliche oder tierische Gefäße einführbar ist und in einem expandierten Zustand in einem Lumen des menschlichen oder tierischen Körpers zum Fördern eines Fluids betreibbar ist, wobei die Katheterpumpe einen Rotor zum Fördern von Fluid, ein den Rotor umgebendes, zumindest teilweise fluiddurchlässiges Gehäuse sowie eine rotornahe Einzugsschleuse aufweist, wobei beim Überführen vom expandierten in den komprimierten Zustand das Gehäuse in die Einzugsschleuse zur zumindest bereichsweisen Durchmesserverengung des Gehäuses einbringbar ist und diese Durchmesserverengung des Gehäuses derart bemessen ist, dass auch der Durchmesser des Rotors durch die Durchmesserverengung des Gehäuses verringert wird.
